# FASCICULE DE BREVET EUROPEEN

(11) **EP 3 362 791 B1**
(45) Date de publication et mention de la délivrance du brevet: **06.03.2024**
(21) Numéro de dépôt: 16781099.3
(22) Date de dépôt: 11.10.2016
(51) Int. Cl.: G01N 33/497, B60K 28/06

(54) **SYSTEME DE CERTIFICATION D'UN DEPISTAGE D'UNE SUBSTANCE GAZEUSE EXHALEE PAR UN INDIVIDU, ET PROCEDE METTANT EN OEUVRE LE SYSTEME**
SYSTEM ZUR ZERTIFIZIERUNG EINER ERFASSUNG EINER GASFÖRMIGEN, VON EINER PERSON AUSGEATMETEN SUBSTANZ UND VERFAHREN ZUR VERWENDUNG DES SYSTEMS
SYSTEM FOR CERTIFYING A DETECTION OF A GASEOUS SUBSTANCE EXHALED BY AN INDIVIDUAL, AND METHOD USING THE SYSTEM

(30) Priorité: 12.10.2015 FR 1559679; 23.06.2016 FR 1655853
(43) Date de publication de la demande: 22.08.2018
(73) Titulaire: Aperli, 75116 Paris (FR)
(72) Inventeur: CADO, Jean-Jacques, 35600 Redon (FR)
(74) Mandataire: Vidon Brevets & Stratégie
(86) Numéro de dépôt international: PCT/EP2016/074268
(87) Numéro de publication internationale: WO 2017/064023

(56) Documents cités:
- EP-A1- 2 075 151
- EP-A1- 2 127 599
- EP-A1- 2 237 034
- US-A1- 2006 237 254
- US-A1- 2013 021 153

## Description

### 1. Domaine de l'invention

L'invention concerne un système de certification d'un dépistage d'une substance gazeuse présente dans l'air exhalé par un individu, le système comprenant un capteur relié à un terminal portable. L'invention concerne notamment le fait que la caméra du terminal prend une image de l'individu faisant le test afin de l'authentifier par reconnaissance faciale.

### 2. Art antérieur

De nos jours l'alcoolisme devient un vrai problème sociétal. En France, on compte 45 000 morts chaque année en lien avec la consommation excessive d'alcool. Celle-ci correspond à la deuxième cause de mortalité évitable dans notre pays. Des drogues telles que le cannabis et l'héroïne sont également la cause de nombreux décès dus à une surconsommation. Les dommages que ces substances provoquent passent quelquefois inaperçus, et ce n'est qu'avec le temps que les effets peu à peu se font sentir, à ce moment il est souvent trop tard. Les substances dites « psychotropes » agissent sur l'état du système nerveux central en y modifiant certains processus biochimiques et physiologiques cérébraux. Les fonctions du cerveau sont altérées en modifiant la perception, les sensations, l'humeur, et de façon générale la conscience et le comportement de celui qui en consomme, c'est pourquoi elles sont nocives.

La société condamne l'utilisation de ces substances psychotropes et notamment lorsqu'elles sont prises avant de prendre le volant. L'une des principales causes de mortalité au volant est l'alcoolisme. Outre les sanctions prévues pour une conduite avec un taux d'alcoolémie supérieur à la législation en vigueur, les effets de l'alcool au volant sont connus : rétrécissement du champ visuel, réduction de la vigilance et de la résistance à la fatigue, diminution des réflexes, altération de la capacité à apprécier les distances, augmentation de la sensibilité à l'éblouissement ou encore excès de confiance en soi. Pour détecter l'absorption de telles substances, les autorités se sont dotées de moyens de détection du taux d'alcoolémie ou d'absorption de certaines drogues. Mais la fréquence des contrôles est faible et rend peu dissuasives ces mesures.

L'alcoolisme au volant peut également être combattu en développant la pratique de l'auto-dépistage. Pour ce faire, les autorités ont récemment obligé les propriétaires à disposer d'au moins un éthylotest dans leur véhicule. Le but d'un éthylotest est de pouvoir indiquer le taux d'alcoolémie contenu dans l'air alvéolaire exhalé dans le souffle de son utilisateur. A noter que ce taux est directement corrélé au taux d'alcoolémie dans le sang. Des modèles d'éthylotest électroniques récemment mis sur le marché sont connectables à un ordiphone (« smartphone » en Anglais) et permettent de mesurer le taux en substances psychotropes exhalées par un individu. La mesure est transmise à l'ordiphone à des fins d'affichage. L'utilisateur de l'ordiphone peut ainsi effectuer son auto-dépistage et décider en toute connaissance de cause de prendre ou non le volant.

Néanmoins, si l'individu ne fait pas le test, ou si le test est effectué mais que son jugement est trop altéré, l'individu peut prendre le volant et se mettre en danger. Si un individu se trouve dans un état de santé non compatible à la conduite automobile, alors on doit l'empêcher par tous les moyens de conduire un véhicule. Cette attitude doit être prise quelque soit l'activité de cet individu, et pas seulement la conduite automobile. Le test peut également être effectué par des pilotes d'avion avant de prendre le décollage. Le test peut aussi être incitatif pour encourager un sevrage. L'éthylotest peut être utilisé par un patient à des fins thérapeutiques afin d'assurer un suivi quotidien de son niveau d'alcoolémie et ainsi l'encourager à ne plus boire.

La demande de brevet US7934577 décrit un mécanisme de contrôle au sein d'un véhicule qui impose au conducteur d'effectuer un auto-dépistage par un éthylotest avant de prendre le volant. Si le résultat du test est positif (des substances psychotropes ont bien été détectées et dans des proportions au-delà d'un seuil) ou si le test n'est pas effectué, alors un dispositif de contrôle empêche le moteur de démarrer. Si le résultat est négatif, alors le dispositif de contrôle autorise l'allumage du moteur. Ce document prévoit que le véhicule comporte une ou plusieurs caméras permettant d'identifier le conducteur. Les caméras et l'éthylotest sont reliés au dispositif de contrôle du véhicule. Cette disposition présente toutefois l'inconvénient qu'il est possible de tricher en faisant souffler le conducteur dans un éthylotest non connecté et un autre individu dans l'éthylotest qui est réellement connecté au dispositif de contrôle du véhicule. Il existe donc un réel besoin d'un système de certification de l'authenticité d'un test de dépistage d'une substance gazeuse appliqué à un individu identifié.

Les documents de brevet n°EP2075151 et n°EP2127599 proposent chacun une solution connue pour détecter si une fraude est commise lors d'un test d'alcoolémie.

Le document US 6 956 848 décrit un système de vérification de l'identité d'un individu consistant en ce que la prise d'image durant le dépistage est comparée à une image déjà prise. Cela ne fonctionne que dans le cas d'un éthylotest anti-démarrage, lorsque l'on connaît les limites de cet appareil. De plus, il est extrêmement difficile de prévoir un système de prise de photo dans le cas d'un éthylotest anti-démarrage. Le document US2006237254 décrit un autre système similaire qui comprend encore des moyens de géolocalisation.

### 3. Objectifs de l'invention

La présente invention apporte une solution qui ne présente pas les inconvénients de l'art antérieur. La solution proposée permet de s'assurer que la mesure du taux de substances psychotropes est associée à un individu dont l'identité est bien reconnue. Cette invention permet notamment d'interdire à cet individu d'effectuer une activité à risque pour lui ou pour les personnes à proximité.

### 4. Exposé de l'invention

La présente invention propose un système de certification selon la revendication 1, ledit système comprend un capteur détectant ladite substance gazeuse dans l'air exhalé. Ledit capteur comprend une unité de recueil et d'analyse d'un échantillon d'air exhalé par ledit individu, apte à fournir une mesure de la quantité de substance gazeuse par unité de volume. Ledit système comprend en outre un terminal portable doté d'un moyen de communication avec le capteur pour au moins recevoir la mesure. Le terminal portable est doté d'une caméra destinée à prendre une image de l'individu soufflant dans ledit capteur pour effectuer un dépistage, et d'un module d'authentification de l'individu et du capteur utilisé pour cet échantillon d'air exhalé en analysant les données d'images par reconnaissance faciale pour l'individu et en analysant un marqueur graphique apposé sur le capteur. Le terminal produit un ensemble d'information comprenant une donnée représentative de la quantité de substance gazeuse mesurée dans l'échantillon et une donnée d'identification authentifiée de l'individu ayant exhalé cet échantillon.

De cette manière, le système peut à la fois authentifier l'identité de la personne qui effectue le test, authentifier le capteur qui est utilisé pour le test, et associer le résultat de la valeur du dépistage à cette personne. Ces données sont utilisables par tout système de contrôle pour autoriser la personne ayant fait le test à effectuer une certaine activité ou à lui interdire si celle-ci peut mettre en danger lui-même ou un groupe d'individus.

Selon un premier mode de réalisation, le système comporte un appareil de contrôle en communication avec le terminal, ledit appareil contrôlant le fonctionnement d'un véhicule et autorisant son démarrage lorsque l'individu identifiée par les données reçues est autorisé à conduire ce véhicule et lorsque la mesure transmise indique que la substance gazeuse n'est pas présente ou en-dessous d'un certain seuil. De cette manière, le véhicule ne peut démarrer que lorsque le dépistage s'est révélé négatif.

Selon un autre mode de réalisation, le système comporte un moyen d'émission sonore intégré dans un véhicule, tel qu'un klaxon, le moyen d'émission est activé lorsque la mesure transmise indique que la substance gazeuse est présente au delà d'un certain seuil. De cette manière, un signal sonore est émis avertissant l'individu et son entourage que le dépistage s'est révélé positif.

Selon un autre mode de réalisation, le système comporte un appareil distant communiquant par radio avec le terminal portable, l'appareil distant émettant vers le terminal portable un signal déclenchant l'apparition d'un menu affiché sur un écran du terminal portable et demandant d'effectuer un dépistage, le terminal portable émettant vers cet appareil distant par le réseau de téléphonie mobile une information représentative de la mesure et la donnée d'identification authentifiée de l'individu. De cette manière, un appareil centralisant des données est mis constamment à jour de l'état des dépistages pratiqués sur les membres d'une organisation.

Selon un autre mode de réalisation, l'appareil distant déclenche une alarme si aucun dépistage ayant abouti à un résultat négatif ne s'est déroulé correctement au cours d'une durée déterminée. De cette manière, le repérage d'un individu n'ayant pas été dépisté depuis un certain temps est facilité.

Le terminal portable dispose d'un moyen de géolocalisation permettant à l'appareil distant de connaître sa position, l'appareil distant émettant vers le terminal portable le signal déclenchant l'apparition d'un menu à la suite d'une immobilisation du terminal au cours d'une durée minimale. De cette manière, si un conducteur fait une halte pour se restaurer par exemple, le système le détecte et lui demande d'effectuer un dépistage.

Selon un autre mode de réalisation, le terminal portable détecte la mise en marche par l'individu d'un appareil à des fins d'utilisation, la mise en marche déclenchant l'apparition du menu demandant à cet individu d'effectuer un dépistage. De cette manière, il est possible de demander un dépistage chaque fois qu'un utilisateur d'une machine la met en marche.

Selon un autre mode de réalisation, ledit capteur comporte un anémomètre détectant le flux d'air résultant d'une exhalation, la détection d'un flux d'air résultant d'une exhalation déclenchant la prise d'au moins une image. De cette manière, la prise d'image est synchronisée à l'exhalation de l'individu ce qui limite les tricheries.

Selon un autre mode de réalisation, le marqueur graphique du capteur est un voyant lumineux visible sur la face opposée à celle d'un embout buccal dans lequel l'individu souffle lors d'un dépistage, le faisceau lumineux émis par ce voyant possède des caractéristiques identifiant ledit capteur. De cette manière, l'authentification du capteur par le terminal est facilitée.

Selon un autre mode de réalisation, au moins une caractéristique du faisceau lumineux émis par le voyant lumineux visible sur la face opposée à celle d'un embout buccal est fournie par le terminal portable via le moyen de communication 9. De cette manière, l'authentification du capteur par le terminal est renforcée, ce qui rend pratiquement impossibles les tricheries en utilisant un autre capteur.

Selon un autre mode de réalisation, le marqueur graphique est un QR Code ou un code à barres apposé sur la face opposée à celle d'un embout buccal. De cette manière, l'authentification du capteur par le terminal est facilitée et nécessite moins de puissance de calcul.

Selon un autre mode de réalisation, le terminal initialise une communication avec le capteur de façon à s'assurer qu'un seul capteur est connecté. De cette manière, la fraude consistant à utiliser au moins deux capteurs est quasi-impossible.

Selon un autre aspect, l'invention concerne un procédé de certification d'un dépistage selon la revendication 13.

Selon un autre aspect, l'invention concerne un produit programme d'ordinateur téléchargeable depuis un réseau de communications et/ou stocké sur un support lisible du système de dépistage précité et/ou exécutable par une unité centrale. Ledit produit programme d'ordinateur comprend des instructions de programme pour la mise en oeuvre d'au moins une étape du procédé de certification d'un dépistage d'une substance gazeuse tel que décrit précédemment et selon l'un quelconque des modes de réalisation.

### 5. Liste des figures

D'autres caractéristiques et avantages de l'invention apparaîtront plus clairement à la lecture de la description suivante d'un mode de réalisation particulier, donné à titre de simples exemples illustratifs et non limitatifs, et des dessins annexés, parmi lesquels :
- la figure 1 représente un système de certification d'un dépistage d'une substance gazeuse selon un exemple de réalisation,
- la figure 2 illustre un mécanisme anti-démarrage intégrant un système de certification d'un dépistage selon un exemple de réalisation,
- la figure 3 représente un exemple d'ordinogramme des principales étapes d'un procédé pour interdire le démarrage d'un véhicule,
- la figure 4 représente une application dans le domaine des transports utilisant un système de certification d'un dépistage,
- la figure 5 présente les principaux composants d'un serveur distant permettant de gérer l'état de dépistage des membres d'une organisation,
- la figure 6 représente un exemple d'ordinogramme des principales étapes d'un procédé de gestion de l'état de dépistage des membres d'une organisation.

### 6. Description d'un mode de réalisation de l'invention

### 6.1 Principe général

L'invention concerne un système de certification d'un dépistage d'une substance gazeuse présente dans l'air exhalé par un individu. Le système comprend un capteur détectant ladite substance gazeuse dans l'air exhalé, ledit capteur comprenant une unité de recueil et d'analyse d'un échantillon d'air exhalé par ledit individu, apte à fournir une mesure de la quantité de substance gazeuse par unité de volume. Le système comprend en outre un terminal portable doté d'un moyen de communication avec le capteur pour au moins recevoir la mesure. Le terminal portable est doté d'une caméra destinée à prendre une image de l'individu soufflant dans ledit capteur pour effectuer un dépistage, et d'un module d'authentification de l'individu et du capteur utilisé pour cet échantillon d'air exhalé en analysant les données d'images par reconnaissance faciale pour l'individu et en analysant un marqueur graphique apposé sur le capteur. Le terminal produit un ensemble d'information comprenant une donnée représentative de la quantité de substance gazeuse mesurée dans l'échantillon et une donnée d'identification authentifiée de cet individu ayant exhalé cet échantillon. De cette manière, le système peut à la fois authentifier l'identité de la personne qui effectue le test, authentifier le capteur qui est utilisé pour le test, et associer le résultat de la valeur du dépistage à cette personne.

### 6.2 Mode particulier de réalisation

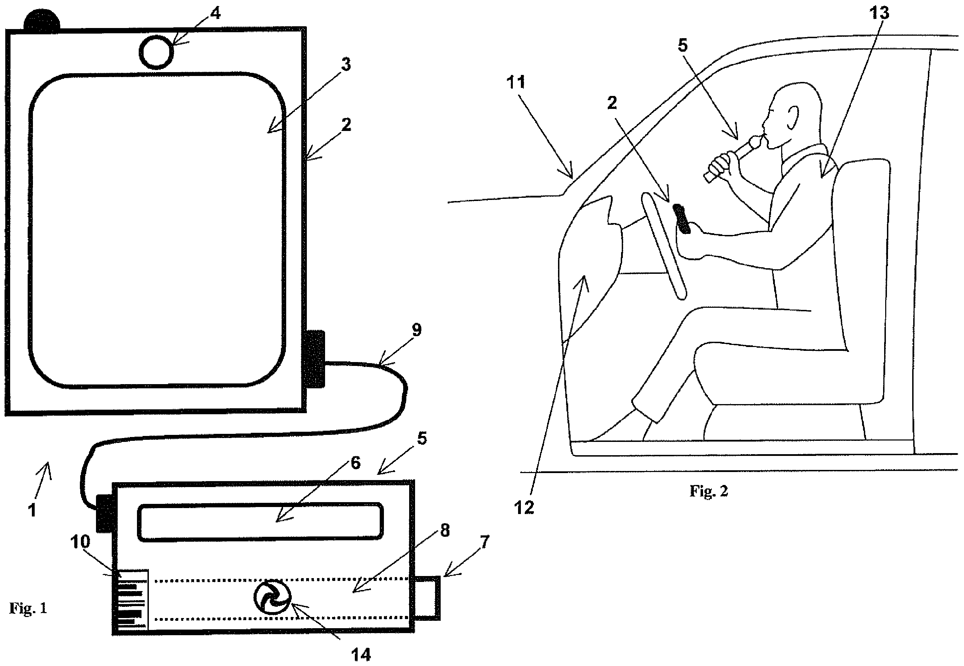

La **Fig. 1** représente un système de certification d'un dépistage d'une substance gazeuse selon un exemple de réalisation. Le système 1 représenté comporte un terminal portable 2 doté d'un moyen de communication avec un réseau, tel qu'un réseau de téléphonie mobile et d'une interface utilisateur 3 telle qu'un écran et un clavier. Cette interface peut comporter en variante, un écran tactile, ou une unité de reconnaissance vocale permettant de recevoir des commandes et d'émettre des informations à un utilisateur. Le terminal 2 comporte un module logiciel de téléchargement d'applications en provenance du réseau de téléphonie et un module d'exécution des applications téléchargées. Le terminal 2 est également doté d'une caméra 4 apte à prendre des photographies ou des vidéos, notamment de son utilisateur qui peut le tenir au bout du bras. Cet utilisateur peut être le conducteur ou tout autre personne, comme une personne majeure accompagnant un mineur au cours d'une conduite accompagnée. Le système comporte également un capteur 5 comprenant une unité de recueil et d'analyse d'un échantillon d'air exhalé par ledit individu, apte à fournir une mesure de la quantité de substance gazeuse par unité de volume. Cette substance gazeuse est de préférence du type psychotrope et couvre notamment l'alcool, le cannabis, l'héroïne. Le capteur 5 peut disposer d'un moyen d'affichage 6. Le capteur 5 comporte un embout 7 permettant à son utilisateur d'exhaler un souffle chargé ou non de ladite substance gazeuse dans un conduit 8 où il est analysé, l'air exhalé s'échappant à une extrémité du conduit 8 opposé à l'embout 7. Le capteur 5 est en communication avec le terminal portable 2 par une liaison informatique 9 filaire ou sans fil (de type Wifi par exemple).

En se connectant à un site distant, le terminal 2 télécharge l'application APLY permettant d'authentifier un dépistage. Une fois téléchargée, l'application APLY active la caméra 4 et le capteur 5 et informe l'utilisateur que le test est prêt à être effectué. Selon un perfectionnement, le terminal 2 initialise une communication avec le capteur 5 de façon à s'assurer qu'un seul capteur est connecté. L'utilisateur oriente l'objectif de la caméra vers lui de façon qu'une partie de son visage apparaisse sur les images prises. Idéalement, au moins une image est prise lorsque l'utilisateur souffle dans l'embout 7, mais ce n'est pas une nécessité, les images peuvent être prises peu de temps avant ou après le test. Le capteur actuellement utilisé nécessite un temps de préchauffage qui peut être mis à profit pour prendre une photographie et identifier la personne. Au moins une partie du visage de l'utilisateur et le capteur 5 sont visibles sur l'image prise. Selon l'invention, le capteur 5 utilisé possède un marqueur graphique 10 permettant de l'identifier parmi tout autre capteur. Ce marqueur est par exemple un code A-barre, ou un QR-code, ou tout symbole graphique apparaissant sur le côté du capteur opposé à celui de l'embout. Ce marqueur peut aussi être un élément lumineux tel qu'une diode LED ou un voyant dont une caractéristique lumineuse au moins identifie le capteur. Par exemple, la diode est commandée par un générateur de PWM dont la fréquence et le rapport cyclique sont particuliers à ce capteur, et ne peuvent être connus d'un autre capteur. Ces signaux lumineux sont captés et analysés par la caméra du terminal. La fréquence et le rapport cyclique sont également transmis par la liaison 9 de façon à être comparés. Avantageusement, les caractéristiques du faisceau lumineux identifiant ledit capteur sont fournies par le terminal portable 2. De cette façon, seul le capteur en communication avec le terminal peut les connaître et on peut s'assurer ainsi qu'un seul capteur est relié à ce terminal.

L'application APLY analyse les données de l'image, ou des images prises pour rechercher un visage et ensuite, pour identifier l'utilisateur par reconnaissance faciale. L'application APLY recherche également la présence du marqueur graphique 10, et en cas de détection, l'analyse pour en extraire l'identifiant. L'analyse de l'image peut s'effectuer au cours d'une étape de préchauffage du capteur et/ou au moment de la mesure. Dans tous les cas, la prise des images et la mesure du capteur s'effectuent à des moments très proches pour s'assurer que la personne photographiée est bien celle qui souffle dans l'éthylotest. Avantageusement, l'écran du terminal 2 affiche des indications permettant d'aider l'utilisateur à effectuer le dépistage. Ces indications sont par exemple :
« Centrez votre visage »,
« Mettez l'embout du capteur à votre bouche »,
« Gardez votre visage bien au centre de l'image et immobile»,
« Tenez le capteur par sa partie basse»,
« Inspirez profondément puis soufflez ».
« Merci ! Le test est terminé.».

Le terminal avertit l'utilisateur du moment où le capteur est opérationnel par un signal lumineux (message affiché sur l'écran 6, allumage du diode LED, éclair de lumière, ...) ou sonore, ou par une vibration. L'apparition de ce signal lumineux et/ou sonore déclenche la prise d'au moins une image avec la caméra 4, pendant ce temps le capteur 5 analyse le souffle exhalé par l'utilisateur et traversant le conduit 8 pour en déterminé le taux en substances psychotropes. Si l'image prise ne permet pas l'identification de l'utilisateur et du capteur, un message apparaît sur l'écran 3 demandant de renouveler le test. Voici les plus fréquentes raisons de l'échec d'un test :
- l'utilisateur n'est pas reconnu,
- le capteur n'est pas reconnu,
- le visage quitte le champ de la caméra,
- d'autres visages sont présents dans l'image,
- la durée du processus est trop longue,
- la qualité de l'image prise est insuffisante (luminosité, contraste, focus)
- la qualité de l'image de référence est insuffisante.

L'application APLY reçoit du capteur 5 via la liaison 9, la donnée représentative de la mesure de la quantité de substance gazeuse par unité de volume, et l'identifiant du capteur. L'application peut alors comparer l'identifiant reçu de celui extrait de l'image du capteur et les comparer. Si les identifiants sont égaux, alors le capteur est authentifié sinon un message d'erreur est affiché. L'application identifie l'utilisateur par reconnaissance faciale et une fois ces vérifications effectuées, associe la mesure avec l'identité de l'utilisateur ayant fait le test. De cette manière, les tricheries concernant l'utilisateur effectuant le test sont pratiquement impossible à réaliser.

Un tel système peut être utilisé par de nombreuses applications. La présente invention prévoit d'intégrer ce système dans un mécanisme d'anti-démarrage d'un véhicule, et dans un procédé de contrôle de l'état des membres d'une organisation.

### 6.3 Application dans un mécanisme d'anti-démarrage

La **Fig. 2** illustre un mécanisme d'anti-démarrage intégrant un système de certification d'un dépistage. Ce mécanisme est présent dans un véhicule 11 qui comporte un ordinateur de bord 12 communiquant notamment avec l'allumage électronique du moteur avec le tableau de fusible du véhicule, ou un brouilleur d'ondes empêchant la propagation de l'onde émise par une clef sans contact. Selon une variante de réalisation, le mécanisme contrôle le klaxon du véhicule et déclenche un effet similaire à celui d'un vol lorsque le dépistage est positif. Par cette communication, l'ordinateur de bord 12 peut interdire le démarrage du moteur lorsque le conducteur 13 utilise sa clef de contact et lorsque certaines conditions sur l'état de santé du conducteur ne sont pas respectées, cet état étant fourni par le système 1 de certification d'un dépistage décrit précédemment. L'ordinateur de bord dispose d'un module de communication avec le terminal portable 2 du conducteur, cette communication s'effectue de préférence par liaison radio à courte portée (en utilisant le « Bluetooth », par exemple), elle peut aussi s'effectuer par une liaison filaire (via un port « USB » par exemple).

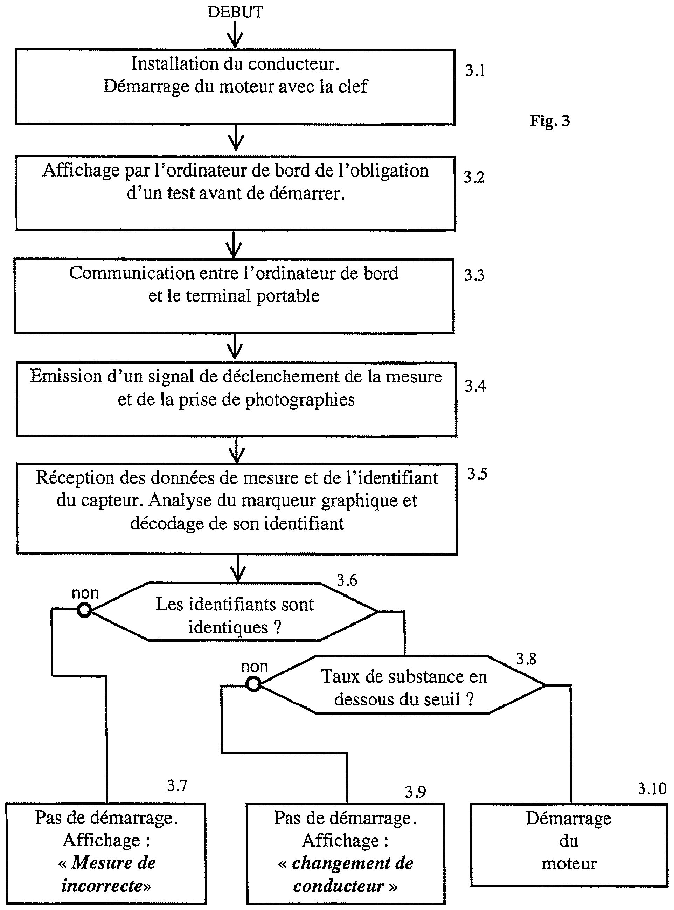

Un mode de réalisation d'un procédé pour interdire le démarrage d'un véhicule est expliqué par l'ordinogramme de la **Fig. 3** donné à titre d'exemple. Cet ordinogramme montre les étapes d'une application utilisant le système 1.

A l'étape 3.1, le conducteur entre dans le véhicule et demande un démarrage avec la clef de contact. L'ordinateur reçoit la commande de démarrage sous la forme d'un signal et informe le conducteur de la nécessité d'effectuer un test de dépistage de substances psychotropes (étape 3.2). Cette information peut s'effectuer par un affichage sur un écran ou par synthèse vocale. L'ordinateur de bord demande alors au conducteur de lancer l'application APLY sur son terminal. A l'étape 3.3, l'ordinateur de bord entre en communication avec le terminal portable 2 et attend les données résultant du test. Le terminal portable active le capteur 5 et prévient le conducteur lorsque le capteur est opérationnel (étape 3.4). En vue de la préparation du test, le terminal informe à l'aide de son écran 3 des conditions de mise en place du test. Ces conditions consistent en ce que la caméra du conducteur est placée face à lui, éventuellement en l'accrochant au rétroviseur central comme le montre la **Fig. 3****,** pour que les données de l'image prise à ce moment comporte à la fois le visage du conducteur et le coté du capteur où se trouve le marqueur graphique.

A l'étape 3.5, le terminal reçoit les données de mesure provenant du capteur et analyse le(s) image(s) prise(s) par la caméra. L'application APLY exécute une tache de reconnaissance faciale afin de déterminer l'identité de la personne présente sur la photo. Cette technologie est connue en soi et permet de fournir une identité avec un maximum de certitude. L'application APLY exécute également un programme de recherche du marqueur graphique présent sur le capteur et de décodage de ce marqueur graphique afin d'en extraire l'identifiant associé au capteur. Le capteur 5 transmet également au terminal 2 le résultat de la mesure et son identifiant par sa liaison 9. Le terminal compare ensuite l'identifiant reçu du capteur du capteur et l'identifiant décodé du marqueur graphique (étape 3.6). Si les identifiants ne sont pas identiques, alors on peut suspecter une tricherie et le terminal 2 émet vers l'ordinateur de bord 12 un signal représentatif d'un échec de la mesure. En recevant ce signal, l'ordinateur n'autorisera pas le démarrage du moteur (étape 3.7). Si par contre, les identités sont respectées alors le souffle analysé est celui de la personne qui est présente devant la caméra 4 et qui tient en main le capteur 5. Dans ce cas, à l'étape 3.8 l'application APLY compare la donnée représentative de la quantité de substance gazeuse par unité de volume avec une valeur de seuil. Le seuil concernant l'alcoolémie est par exemple de 0,5 grammes par litre de sang. Si le seuil est dépassé, le terminal 2 émet vers l'ordinateur de bord 12 un signal indiquant que l'identité du conducteur est vérifiée mais qu'il n'est pas dans un état lui permettant de conduire. Un message du type « changer de conducteur » peut être reproduit (étape 3.9). Si par contre, le seuil d'alcoolémie n'est pas dépassé et si aucune drogue n'est détectée, l'ordinateur autorise le démarrage du moteur (étape 3.10).

L'application ainsi décrite permet de s'assurer avec un haut niveau de sécurité que le conducteur a bien effectué le test. Selon une technique connue en soi, le capteur 5 possède un micro-anémomètre 14 intégré dans le conduit 8 qui contrôle notamment que le débit d'air exhalé et que son volume est suffisant, en effet seul l'air alvéolaire est chargé en alcool et il faut souffler sans discontinuer un certain volume pour l'exhaler. Selon un perfectionnement facultatif, dès que le micro-anémomètre détecte la présence d'un souffle, le capteur émet vers le terminal 2 un signal de début de mesure qui déclenche la prise d'au moins une première image. Lorsque le micro-anémomètre détecte la fin du souffle, la mesure est terminée et la valeur de mesure du taux de substances psychotrope est transmise au terminal.

### 6.4 Application dans un mécanisme de contrôle de l'état de dépistage des membres d'une organisation

Le système de certification d'un dépistage d'une substance gazeuse peut également être utilisé pour contrôler l'état de dépistage de membres d'une organisation, et ceci avec un haut niveau de sécurité. Le terme « organisation » décrit tout groupe d'individus chargés d'effectuer un certain travail nécessitant un bon niveau de santé et sous la responsabilité d'une autorité. Une organisation est par exemple une société de transport où des conducteurs transportant des passagers effectuent des trajets. Cela peut aussi des ouvriers dans une usine, des laveurs de carreaux, des maçons, etc ... de façon générale toute profession pour laquelle un salarié doit être en pleine possession de ses moyens.

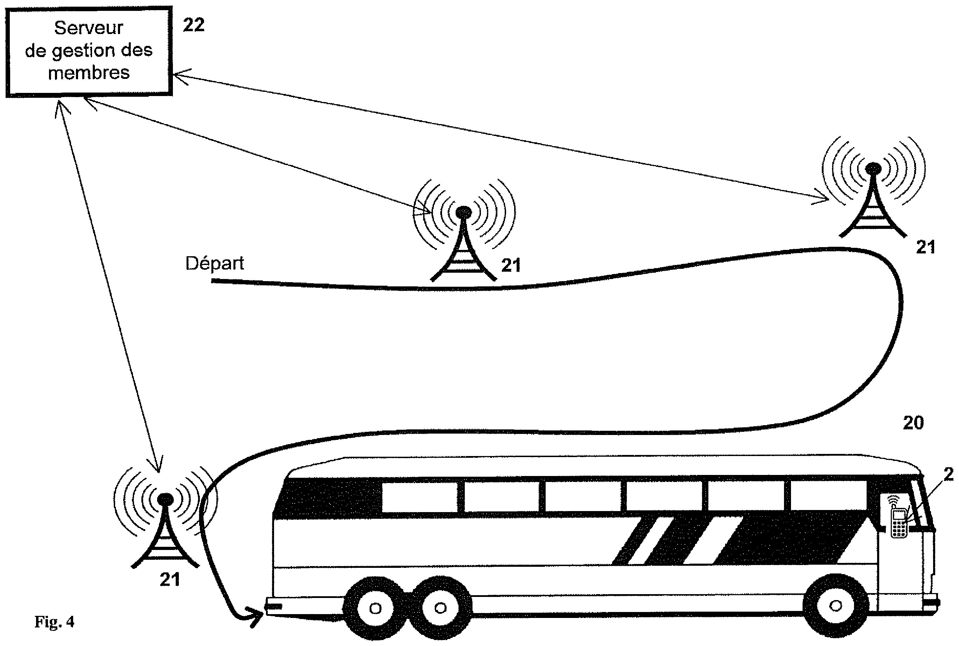

La **Fig. 4** illustre une telle application dans le domaine des transports, par exemple des cars 20 transportant des passagers. Au cours de son trajet, le car 20 traverse des aires géographiques couvertes par des réseaux téléphoniques représentés par des stations fixes 21, appartenant à un opérateur de téléphonique mobile du type 3G/4G par exemple. Le conducteur du car est doté du système 1 décrit précédemment. Le terminal portable 2 entre en communication avec les différentes stations fixes 21 et possède un moyen de géolocalisation. Ce moyen peut être le réseau téléphonique lui-même en analysant par triangulation les temps de retours des communications avec plusieurs bases fixes. Ce moyen peut également être un module GPS (Global Positioning System) intégré dans le terminal. Dans tous les cas, la localisation du terminal, et par voie de conséquence celle du conducteur du car, est transmise via le réseau téléphonique à un serveur distant, dit « de gestion des membres de l'organisation », ou serveur « GMO » 22.

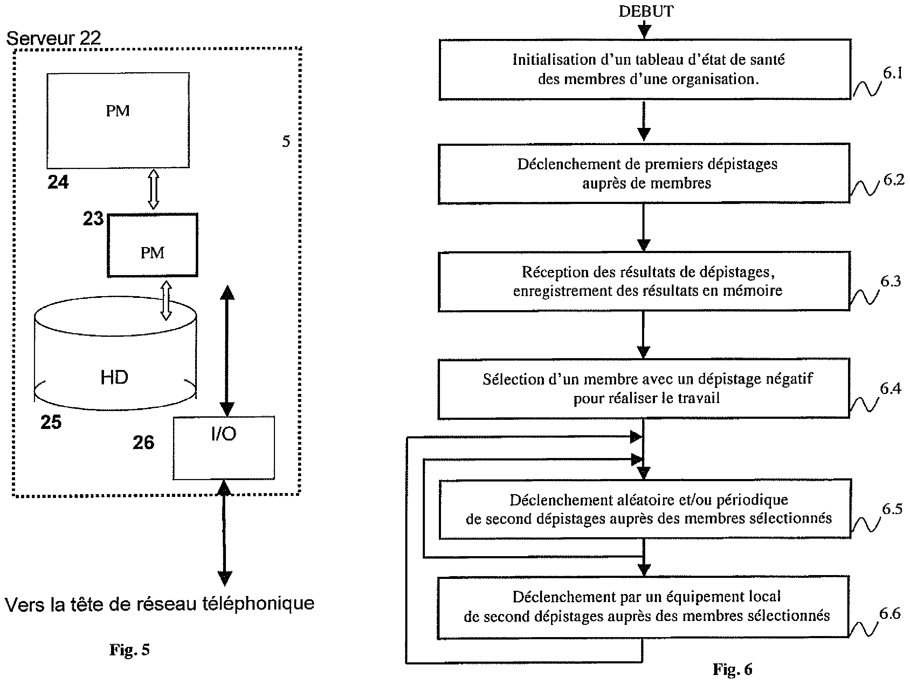

La **Fig. 5** illustre les principaux composants d'un serveur GMO 22. Selon cet exemple de réalisation, le serveur GMO 22 comporte une unité centrale ALU 23 reliée à une mémoire de programme exécutable PM 24, un disque dur HD 25 contenant une base de données pour le stockage de données de façon non-volatile. La mémoire de programme comporte une application GMO permettant de gérer les membres de l'organisation et de connaître au moins les derniers dépistages qui ont été effectués sur eux. Le serveur 22 contient également une interface I/O 26 pour la communication avec le réseau téléphonique. Les messages reçus par l'interface I/O permettent de recevoir des données sur le dépistage effectuées sur les différents conducteurs réalisant des trajets. Il n'est pas exclu que les moyens de liaison diffèrent selon le type d'appareil en communication, ainsi le serveur d'optimisation 23 peut communiquer avec les téléphones à travers un réseau sans fil (téléphonie mobile 3G/4G) ou par un câble via un réseau numérique quelconque (Internet par exemple).

Après avoir détaillé les principaux éléments constitutifs de cette application, nous allons maintenant expliquer comment ceux-ci coopèrent.

Un mode de réalisation d'une application de gestion de l'état de santé de membres est expliqué par l'ordinogramme de la **Fig. 6** qui illustre un exemple préféré de réalisation. L'exemple d'ordinogramme montre les étapes d'un exemple de mise en oeuvre du procédé selon l'invention.

Lors d'une étape préalable 6.1, le serveur 23 initialise dans sa mémoire 25 un tableau comprenant des lignes correspondant à chaque membre de l'organisation. Chaque ligne du tableau comporte les informations suivantes :
- Identité (nom et prénom) du membre et numéro de téléphone,
- données biométriques identifiant le visage du membre,
- date et heure du dernier test,
- état du dernier test.

A un moment donné un besoin en personnel apparaît et une étape de recherche d'au moins un membre apte à effectuer un certain travail est lancée. L'application GMO appelle alors une sous liste de membres et leur demande d'effectuer le test de dépistage (étape 6.2). Le test s'effectue en utilisant le système décrit au paragraphe 6.2. Dès que les premiers résultats arrivent au niveau du serveur, ils sont enregistrés dans la mémoire 25 (étape 6.3). Avantageusement, la localisation du lieu du dépistage est également enregistrée. A l'étape 6 .4, au moins un membre est sélectionné pour le travail à effectuer. Ces membres sélectionnés doivent bien sûr avoir réalisé un dépistage négatif. Les membres sélectionnés sont ensuite contactés pour leur donner les directives à suivre afin d'effectuer le travail demandé.

Selon un perfectionnement, au cours de la réalisation du travail, le membre est contacté par son terminal portable 2 pour effectuer un nouveau dépistage (étape 6.5). Les déclenchements de ces nouveaux dépistages peuvent s'effectuer à des intervalles de temps réguliers, toutes les 3 heures par exemple. Selon une variante, le déclenchement intervient aléatoirement dans le temps, par exemple dans une fourchette de temps entre une heure et 6 heures à compter du précédent test.

Si un nouveau test ne peut s'effectuer, il existe donc un doute sur l'état de dépistage de ce membre, alors le serveur peut déclencher une alarme dans le but de remplacer ce membre par un autre, cet autre ayant préalablement subi un dépistage négatif.

Selon un perfectionnement, si le dépistage est positif, alors un message est transmis à au moins une tierce personne dont l'identité est enregistrée dans le serveur 23. Le message peut être transmis par tout moyen : SMS, courriel, appel vocal par téléphone, ... Cette personne peut être un parent (dans le cadre d'un monitoring familial par exemple), soit à un responsable hiérarchique ou un agent de sécurité (dans le cadre du travail et du dépistage à distance), soit un conseiller médical ou un professionnel de santé.

Selon un mode de réalisation en accord avec l'invention et qui s'applique prioritairement à une organisation dans le domaine du transport, le serveur GMO suit en temps réel le déplacement des membres et détermine si ceux-ci sont mobiles ou fixes. Le déclenchement d'un nouveau dépistage s'effectue lorsque le membre ne se déplace pas dans son véhicule, ou en d'autres termes si sa vitesse de déplacement est inférieure à 5 kilomètres heure par exemple. Au delà de cette valeur, on peut considérer qu'il est au volant et que ce n'est pas le moment de le déranger. Selon ce mode de réalisation, le test s'effectue lorsque le serveur GMO détecte une immobilisation pendant au moins un certain temps, puis un déplacement inférieur à une certaine vitesse (typiquement 5 Km/h, qui peut signifier qu'après un arrêt, le chauffeur regagne à pied son véhicule). On peut supposer qu'il a fait une pause déjeuner et que c'est le moment de vérifier qu'il n'a pas consommé de l'alcool.

Selon une variante de réalisation, le terminal 2 reçoit par radio à courte portée des signaux provenant d'émetteurs locaux, ces signaux déclenchant le dépistage (étape 6.6). Cette variante intervient par exemple lors de l'activation de la clef de contact d'un véhicule, ou la mise en route de la machine sur laquelle un ouvrier travaille. Cette variante peut également intervenir lorsque des caméras de surveillance détectent le comportement inhabituel d'un salarié. Selon cette variante, une fois le dépistage effectué, le terminal 2 transmet au serveur GMO les résultats à des fins d'enregistrements dans la mémoire 25. Cette variante est parfaitement combinable avec le fait que le serveur demande également d'effectuer des dépistages. Dans ce cas, le serveur GMO enregistre dans la mémoire 25 l'équipement qui a déclenché le dépistage : soit un équipement local, soit le serveur.

### 6.5 Application pour gérer des dossiers médicaux

Le système de certification d'un dépistage d'une substance gazeuse peut également être utilisé pour mettre à jour des données médicales délocalisées. Les patients sont évités à effectuer des dépistages à certains moments et les valeurs ainsi mesurées sont transmises par le réseau téléphonique et enregistrées dans un serveur distant. Selon les patients, les demandes de dépistages sont émises à des moments aléatoires au cours d'une période donnée, ou à heures fixes, ou encore au cours des périodes de « craving » de ce patient (c'est à dire, à des moments où il a une irrépressible envie de boire). le serveur est consultable par des professionnels de santé qui peuvent prendre connaissance du taux en substances psychotropes exhalées par un patient, et ainsi voir l'évolution au fil du temps. Cette évolution est particulièrement utile lors d'un sevrage, notamment pour aider une personne à ne plus être dépendante de l'alcool. L'accès aux données du serveur est sécurisé de façon que seuls des professionnels de santé et/ou le patient lui-même peuvent lire les données enregistrées. Le fait d'autoriser le patient à accéder aux données est une aide car cela lui permet de voir que ses efforts procure un résultat. En fonction de la valeur de taux détecté, des messages sont envoyés aux personnes, soit des messages de félicitations si les valeurs sont basses, soit des messages d'encouragement, soit des messages l'incitant à prendre contact avec un professionnel de santé.

L'invention n'est pas limitée aux modes de réalisation qui viennent d'être décrits. En particulier, le système est utilisable pour toute activité humaine où il est nécessaire de s'assurer que la mesure du taux de substances psychotropes associée à un individu est bien inférieure à un seuil déterminé.

## Revendications

1. Système de certification d'un dépistage d'une substance gazeuse présente dans l'air exhalé par un individu, ledit système comprenant un capteur (5) détectant ladite substance gazeuse dans l'air exhalé, ledit capteur comprenant une unité de recueil et d'analyse d'un échantillon d'air exhalé par ledit individu, apte à fournir une mesure de la quantité de substance gazeuse par unité de volume, ledit système comprenant en outre un terminal portable (2) doté d'un moyen de communication (9) avec le capteur pour au moins recevoir la mesure, d'une caméra (4) destinée à prendre une image de l'individu soufflant dans ledit capteur pour effectuer un dépistage, et d'un module d'authentification de l'individu et du capteur utilisé pour cet échantillon d'air exhalé en analysant les données d'images par reconnaissance faciale pour l'individu et en analysant un marqueur graphique apposé sur le capteur, le terminal (2) produisant un ensemble d'informations comprenant une donnée représentative de la quantité de substance gazeuse mesurée dans l'échantillon et une donnée d'identification authentifiée de l'individu ayant exhalé cet échantillon,
le système comportant un appareil distant (22) communiquant par radio avec le terminal portable (2), l'appareil distant (22) émettant vers le terminal portable (2) un signal déclenchant l'apparition d'un menu affiché sur un écran (3) du terminal portable et demandant d'effectuer un dépistage, le terminal portable (2) émettant vers cet appareil distant (22) par un réseau de téléphonie mobile une information représentative de la mesure et la donnée d'identification authentifiée de l'individu, le terminal portable (2) disposant d'un moyen de géolocalisation permettant à l'appareil distant (22) de connaître sa position, **caractérisé en ce que** l'appareil distant (22) émet vers le terminal portable(2) le signal déclenchant l'apparition d'un menu à la suite d'une immobilisation du terminal au cours d'une durée minimale puis d'un déplacement inférieur à une vitesse prédéfinie.

2. Système selon la revendication 1, **caractérisé en ce qu'**il comporte un appareil de contrôle en communication avec le terminal (2), ledit appareil contrôlant le fonctionnement d'un véhicule et autorisant son démarrage lorsque l'individu identifiée par les données reçues est autorisé à conduire ce véhicule et lorsque la mesure transmise indique que la substance gazeuse n'est pas présente ou en-dessous d'un certain seuil.

3. Système selon la revendication 1, **caractérisé en ce qu'**il comporte un moyen d'émission sonore intégré dans un véhicule, tel qu'un klaxon, le moyen d'émission est activé lorsque la mesure transmise indique que la substance gazeuse est présente au-delà d'un certain seuil.

4. Système selon l'une quelconque des revendications 1 à 3, **caractérisé en ce que** l'appareil distant (22) déclenche une alarme si aucun dépistage n'ayant abouti à un résultat négatif ne s'est déroulé correctement au cours d'une durée déterminée.

5. Système selon l'une quelconque des revendication 1 à 4, **caractérisé en ce que** le terminal portable (2) détecte la mise en marche par l'individu d'un appareil à des fins d'utilisation, la mise en marche déclenchant l'apparition du menu demandant à cet individu d'effectuer un dépistage.

6. Système selon l'une quelconque des revendications précédentes, **caractérisé en ce que** ledit capteur (5) comporte un anémomètre (14) détectant le flux d'air résultant d'une exhalation, la détection d'un flux d'air résultant d'une exhalation déclenchant la prise d'au moins une image.

7. Système selon l'une quelconque des revendications précédentes, **caractérisé en ce que** le marqueur graphique du capteur (5) est un voyant lumineux visible sur la face opposée à celle d'un embout buccal (7) dans lequel l'individu souffle lors d'un dépistage, le faisceau lumineux émis par ce voyant possède des caractéristiques identifiant ledit capteur.

8. Système selon la revendication 7, **caractérisé en ce qu'**au moins une caractéristique du faisceau lumineux émis par le voyant lumineux visible sur la face opposée à celle d'un embout buccal (7) est fournie par le terminal portable (2) via le moyen de communication (9).

9. Système selon l'une quelconque des revendications 1 à 6, **caractérisé en ce que** le marqueur graphique est un QR Code ou un code à barres apposé sur la face opposée à celle d'un embout buccal (7).

10. Système selon l'une quelconque des revendications précédentes, **caractérisé en ce que** le terminal (2) initialise une communication avec le capteur (5) de façon à s'assurer qu'un seul capteur est connecté.

11. Système selon l'une quelconque des revendications précédentes, **caractérisé en ce que** le dit appareil distant enregistre les mesures effectuées au fil du temps, et accorde un accès sécurisé à ces mesures pour un professionnel de santé et/ou à l'utilisateur lui-même.

12. Système selon l'une quelconque des revendications précédentes, **caractérisée en ce que** le dit appareil distant dispose d'un moyen d'alerte vers un professionnel de santé ou toute autre autorité en cas de non-dépistage ou de dépistage positif d'un utilisateur sous sa dépendance.

13. Procédé de certification d'un dépistage d'une substance gazeuse présente dans l'air exhalé par un individu en utilisant un capteur (5) détectant ladite substance gazeuse dans l'air exhalé, ledit procédé comportant les étapes suivantes :
- exhalation par ledit individu dans ledit capteur afin de fournir une mesure de la quantité de substance gazeuse par unité de volume,
- prise d'une image au moins de l'individu soufflant dans ledit capteur pour effectuer un dépistage à l'aide de la caméra (4) d'un terminal portable (2) connecté audit capteur (5),
- authentification de l'individu et du capteur utilisé pour cet échantillon d'air exhalé en analysant les données d'images par reconnaissance faciale pour l'individu et en analysant un marqueur graphique apposé sur le capteur,
- production par le terminal (2) d'un ensemble d'informations comprenant une donnée représentative de la quantité de substance gazeuse mesurée dans l'échantillon et une donnée d'identification authentifiée de l'individu ayant exhalé cet échantillon, le terminal comportant encore des moyens de géolocalisation,
le procédé étant **caractérisé en ce qu'**il comprend une émission vers le terminal portable (2) d'un signal déclenchant l'apparition d'un menu affiché sur un écran (3) du terminal portable et demandant d'effectuer un dépistage, effectuée à la suite d'une immobilisation du terminal au cours d'une durée minimale puis d'un déplacement inférieur à une vitesse prédéfinie.

14. Produit programme d'ordinateur téléchargeable depuis un réseau de communications et/ou stocké sur un support lisible du système de certification selon les revendications 1 à 12, **caractérisé en ce qu'**il comprend les instructions de programme pour la mise en oeuvre du procédé de certification d'un dépistage d'une substance gazeuse présente dans l'air exhalé par un individu selon la revendication 13, lors-qu'il sont exécutée par un système selon les revendications 1 à 12.

## Patentansprüche

1. System zur Zertifizierung einer gasförmigen, von einer Person ausgeatmeten Substanz, wobei das System einen Sensor (5) umfasst, der die gasförmige Substanz in der ausgeatmeten Luft erfasst, wobei der Sensor eine Einheit zum Sammeln und Analysieren einer Probe von der Person ausgeatmeter Luft umfasst, die geeignet ist, eine Messung der Menge an gasförmiger Substanz pro Volumeneinheit zu liefern, wobei das System ferner ein tragbares Endgerät (2) umfasst, das mit einem Mittel (9) zur Kommunikation mit dem Sensor, um zumindest die Messung zu erhalten, einer Kamera (4), die dazu bestimmt ist, eine Aufnahme von der in den Sensor atmenden Person zu machen, um eine Erfassung durchzuführen, und einem Modul zur Authentifizierung der Person und des für diese Probe an ausgeatmeter Luft verwendeten Sensors versehen ist, wobei die Daten von Aufnahmen durch Gesichtserkennung für die Person analysiert werden und ein auf dem Sensor angebrachter Marker analysiert wird, wobei das Endgerät (2) eine Gesamtheit von Informationen erzeugt, umfassend ein Datum, das für die Menge an in der Probe gemessener gasförmiger Substanz repräsentativ ist, und ein Datum zur authentifizierten Identifikation der Person, die diese Probe ausgeatmet hat, wobei das System ein entferntes Gerät (22) umfasst, das per Funk mit dem tragbaren Endgerät (2) kommuniziert, wobei das entfernte Gerät (22) zu dem tragbaren Endgerät (2) ein Signal sendet, das das Erscheinen eines auf einem Bildschirm (3) des tragbaren Endgeräts angezeigten Menüs auslöst und dazu auffordert, eine Erfassung durchzuführen, wobei das tragbare Endgerät (2) zu diesem entfernten Gerät (22) per Mobiltelefonnetz eine Information sendet, die für die Messung und das Datum zur authentifizierten Identifikation der Person repräsentativ ist, wobei das tragbare Endgerät (2) über ein Mittel zur Geolokalisierung verfügt, das es dem entfernten Gerät (22) ermöglicht, seine Position zu erkennen, **dadurch gekennzeichnet, dass** das entfernte Gerät (22) zu dem tragbaren Endgerät (2) das Signal sendet, das das Erscheinen eines Menüs nach einer Immobilisierung des Endgeräts während einer Mindestdauer und dann einer Verlagerung unter einer vordefinierten Geschwindigkeit auslöst.

2. System nach Anspruch 1, **dadurch gekennzeichnet, dass** es ein Kontrollgerät, das mit dem Endgerät (2) kommuniziert, umfasst, wobei das Gerät die Funktion eines Fahrzeugs kontrolliert und seinen Start gestattet, wenn die durch die empfangenen identifizierte Person autorisiert ist, dieses Fahrzeug zu lenken, und wenn die übertragene Messung zeigt, dass die gasförmige Substanz nicht vorhanden ist oder unter einem bestimmten Grenzwert liegt.

3. System nach Anspruch 1, **dadurch gekennzeichnet, dass** es ein in das Fahrzeug integriertes akustisches Sendemittel, wie eine Hupe, umfasst, wobei das Sendemittel aktiviert wird, wenn die übertragene Messung zeigt, dass sie gasförmige Substanz über einem gewissen Grenzwert vorhanden ist.

4. System nach einem der Ansprüche 1 bis 3, **dadurch gekennzeichnet, dass** das entfernte Gerät (22) einen Alarm auslöst, wenn keine Erfassung, die nicht zu einem negativen Ergebnis geführt hat, korrekt während einer bestimmten Dauer stattgefunden hat.

5. System nach einem der Ansprüche 1 bis 4, **dadurch gekennzeichnet, dass** das tragbare Endgerät (2) das Ingangsetzen eines Geräts zu Verwendungszwecken durch die Person erfasst, wobei das Ingangsetzen das Erscheinen des Menüs auslöst, das diese Person auffordert, eine Erfassung durchzuführen.

6. System nach einem der vorhergehenden Ansprüche, **dadurch gekennzeichnet, dass** der Sensor (5) einen Windmesser (14) umfasst, der den aus einem Ausatmen stammenden Luftstrom erfasst, wobei sich die Erfassung eines Luftstroms aus einem Ausatmen ergibt, das die Aufnahme eines Bildes auslöst.

7. System nach einem der vorhergehenden Ansprüche, **dadurch gekennzeichnet, dass** der Grafikmarker des Sensors (5) eine Kontrollleuchte ist, die auf der entgegengesetzten Seite zu jener eines Mundstücks (7), in das die Person während einer Erfassung bläst, sichtbar ist, wobei der von dieser Leuchte ausgesandte Lichtstrahl Merkmale besitzt, die den Sensor identifizieren.

8. System nach Anspruch 7, **dadurch gekennzeichnet, dass** mindestens ein Merkmal des Lichtstrahls, der von der Kontrollleuchte ausgesandt wird, die auf der entgegengesetzten Seite zu jener eines Mundstücks (7) sichtbar ist, von dem tragbaren Endgerät (2) über das Kommunikationsmittel (9) geliefert wird.

9. System nach einem der Ansprüche 1 bis 6, **dadurch gekennzeichnet, dass** der Grafikmarker ein QR-Code oder ein Barcode ist, der auf der zu jener eines Mundstücks (7) entgegengesetzten Seite angebracht ist.

10. System nach einem der vorhergehenden Ansprüche, **dadurch gekennzeichnet, dass** das Endgerät (2) eine Kommunikation mit dem Sensor (5) initialisiert, um sich zu vergewissern, dass nur ein Sensor angeschlossen ist.

11. System nach einem der vorhergehenden Ansprüche, **dadurch gekennzeichnet, dass** das entfernte Gerät die Messungen, die im Laufe der Zeit durchgeführt wurden, aufzeichnet und einen sicheren Zugang zu diesen Messungen für eine medizinische Fachperson und/oder den Benutzer selbst gewährt.

12. System nach einem der vorhergehenden Ansprüche, **dadurch gekennzeichnet, dass** das entfernte Gerät über ein Warnmittel zu einer medizinischen Fachperson oder jeder anderen Autorität im Falle einer Nichterfassung oder einer positiven Erfassung eines Benutzers unter seiner Abhängigkeit verfügt.

13. Verfahren zur Zertifizierung einer Erfassung einer gasförmigen von einer Person ausgeatmeten Substanz unter Verwendung eines Sensors (5), der die ausgeatmete gasförmige Substanz erfasst, wobei das Verfahren die folgenden Schritte umfasst:
- Ausatmen durch die Person in den Sensor, um eine Messung der Menge an gasförmiger Substanz pro Volumeneinheit zu liefern,
- Aufnehmen eines Bildes von zumindest der Person, die in den Sensor ausatmet, um eine Erfassung mit Hilfe der Kamera (4) eines an den Sensor (5) angeschlossenen tragbaren Endgeräts (2) durchzuführen,
- Authentifizierung der Person und des für diese ausgeatmete Luftprobe verwendeten Sensors durch Analyse der Bilddaten durch Gesichtserkennung für die Person und durch Analyse eines auf dem Sensor angebrachten Grafikmarkers,
- durch das Endgerät (2) Erzeugung einer Gesamtheit von Informationen, umfassend ein Datum, das für die Menge an in der Probe gemessener gasförmiger Substanz repräsentativ ist, und ein Datum zur authentifizierten Identifikation der Person, die diese Probe ausgeatmet hat, wobei das Endgerät ferner Mittel zur Geolokalisierung umfasst,
wobei das Verfahren **dadurch gekennzeichnet ist, dass** ein Senden eines Signals zu dem tragbaren Endgerät (2) umfasst, welches das Erscheinen eines auf einem Bildschirm (3) des tragbaren Endgeräts angezeigten Menüs auslöst und dazu auffordert, eine Erfassung durchzuführen, die nach einer Immobilisierung des Endgeräts während einer Mindestdauer und dann einer Verlagerung unter einer vordefinierten Geschwindigkeit erfolgt.

14. Computerprogrammprodukt, das von einem Kommunikationsnetz heruntergeladen werden kann und/oder auf einem lesbaren Träger des Zertifizierungssystems nach den Ansprüchen 1 bis 12 gespeichert sein kann, **dadurch gekennzeichnet, dass** es die Programmbefehle für den Einsatz des Verfahrens zur Zertifizierung einer Erfassung einer gasförmigen von einer Person ausgeatmeten Substanz nach Anspruch 13 umfasst, wenn es von einem System nach den Ansprüchen 1 bis 12 eingesetzt wird.

## Claims

1. System for certifying a detection of a gaseous substance present in the air exhaled by an individual, said system comprising a sensor (5) detecting said gaseous substance in the exhaled air, said sensor comprising a unit for collecting and analysing a sample of air exhaled by said individual, capable of providing a measurement of the quantity of gaseous substance per unit of volume, said system further comprising a portable terminal (2) equipped with a communication means (9) for communicating with the sensor so as to at least receive the measurement, a camera (4) intended to capture an image of the individual blowing into this sensor to perform a detection, and with a module for authenticating the individual and the sensor used for said exhaled air sample by analysing the data of images using facial recognition for the individual and by analysing a graphic marker placed on this sensor, the terminal (2) producing a set of information comprising an item of data representative of the quantity of gaseous substance measured in the sample and an authenticated identification data of the individual who has exhaled said sample, the system comprising a remote device (22) communicating by radio with the portable terminal (2), the remote device (22) emitting a signal to the portable terminal (2), this signal triggering the appearance of a menu displayed on a screen (3) of the portable terminal and requesting the completion of a test, the portable terminal (2) emitting an information item to said remote device (22) via the mobile phone network, this information item being representative of the measurement and the authenticated identification data of the individual, the portable terminal (2) has a geolocation means allowing the remote device (22) to obtain the position thereof **characterized in that** the remote device sends to the portable terminal this signal triggering the appearance of a menu after an immobilisation of the terminal for a minimum period of time then a movement at less than a given speed.

2. System according to claim 1, **characterized in that** it comprises a control device in communication with the terminal (2), said device controlling the operation of a vehicle and authorising start-up thereof if the individual identified by the data received is authorised to drive said vehicle and if the transmitted measurement shows that the gaseous substance is not present or is present below a certain threshold.

3. System according to claim 1, **characterized in that** it comprises a means for emitting a sound integrated into a vehicle, such as a horn, the emitting means being activated when the transmitted measurement shows that the gaseous substance is present beyond a certain threshold.

4. System according to any one of claims 1 to 3, **characterized in that** the remote device (22) triggers an alarm if no test producing a negative result has been correctly carried out during a given period of time.

5. System according to any of claims 1 to 4, **characterized in that** the portable terminal (2) detects the activation by the individual of a device for the use thereof, the activation triggering the appearance of the menu requesting said individual to carry out a test.

6. System according to any of the previous claims, **characterized in that** said sensor (5) comprises an anemometer (14) detecting the airflow resulting from an exhalation, the detection of an airflow resulting from an exhalation triggering the capture of at least one image.

7. System according to any of the previous claims, **characterized in that** the graphic marker of the sensor (5) is a visible indicator light on the side opposite that of a mouthpiece (7) into which the individual blows when carrying out a test, the light beam emitted by said indicator having features that identify said sensor.

8. System according to claim 7, **characterized in that** at least one feature of the light beam emitted by the visible indicator light on the side opposite that of a mouthpiece (7) is provided by the portable terminal (2) via the communication means (9).

9. System according to any of claims 1 to 6, **characterized in that** the graphic marker is a QR Code or a barcode placed on the side opposite that of a mouthpiece (7).

10. System according to any of the previous claims, **characterized in that** the terminal (2) initialises communication with the sensor (5) in order to ensure that a single sensor is connected.

11. System according to any of the previous claims, **characterized in that** said remote device progressively records the measurements taken overtime, and provides secure access to said measurements for a healthcare professional and/or for the user.

12. System according to any of the previous claims, **characterized in that** said remote device is provided with a means for alerting a healthcare professional or any other authority in the event that no detection is carried out or if positive detection results are obtained by a user dependent thereon.

13. Method for certifying a detection of a gaseous substance present in the air exhaled by an individual using a sensor (5) detecting said gaseous substance in the exhaled air, said method comprising the steps of:
- exhaling by said individual into the sensor in order to provide a measurement of the quantity of gaseous substance per unit of volume,
- capturing an image of at least the individual blowing into said sensor in order to carry out a test using the camera (4) of a portable terminal (2) connected to said sensor (5),
- authenticating the individual and the sensor used for said exhaled air sample by analysing the data of images using facial recognition for the individual and by analysing a graphic marker placed on the sensor,
- producing, by the terminal (2), a set of information comprising an item of data representative of the quantity of gaseous substance measured in the sample and an authenticated identification data of the individual who has exhaled said sample, the terminal comprising also geolocation means,
the method being **characterized in that** it comprises an emission to the portable terminal of a signal triggering the appearance of a menu on screen of the portable terminal and asking to perform a detection, carried out after an immobilisation of the terminal for a minimum period of time then a movement at less than a given speed.

14. Computer program product available for download from a communication network and/or stored on a computer-readable medium of the system for certifying according to claims 1 to 12, **characterized in that** it comprises program instructions for implementing the method for certifying a detection of a gaseous substance present in the air exhaled by an individual according to claim 13, when executed by a system according to claims 1 to 12.
